# EUROPÄISCHE PATENTSCHRIFT

(11) **EP 2 056 723 B1**
(45) Veröffentlichungstag und Bekanntmachung des Hinweises auf die Patenterteilung: **28.07.2010**
(21) Anmeldenummer: 07785937.9
(22) Anmeldetag: 07.07.2007
(51) Int. Cl.: A61B 17/00

(54) **VERSCHLUSSELEMENT FÜR ÖFFNUNGEN IM HERZEN, INSBESONDERE ASD-VERSCHLUSSELEMENT**
OCCLUSION ELEMENT FOR OPENINGS IN THE HEART, IN PARTICULAR AN ASD OCCLUSION ELEMENT
ÉLÉMENT D'OBTURATION POUR DES ORIFICES DANS LE COEUR HUMAIN, NOTAMMENT ÉLÉMENT D'OBTURATION DE CIA (COMMUNICATION INTERAURICULAIRE)

(30) Priorität: 29.08.2006 DE 102006040415
(43) Veröffentlichungstag der Anmeldung: 13.05.2009
(73) Patentinhaber: Peter Osypka Stiftung Stiftung des bürgerlichen Rechts, 79639 Grenzach-Wyhlen (DE)
(72) Erfinder: OSYPKA, Peter, 79618 Rheinfelden-Herten (DE)
(74) Vertreter: Maucher, Wolfgang
(86) Internationale Anmeldenummer: PCT/EP2007/006033
(87) Internationale Veröffentlichungsnummer: WO 2008/025405

(56) Entgegenhaltungen:
- DE-B3- 10 338 702

## Beschreibung

Die Erfindung betrifft ein Verschlusselement für ungewollte Öffnungen im Herzen, insbesondere ein ASD-Verschlusselement oder Occlusionsinstrument, welches aus Memory-Material-Fäden, insbesondere aus Memory-Kunsstoffäden, aus Nitinoldrähten oder aus mit Kunststoff ummantelten Drähten oder Fäden aus Memory-Material, geflochten ist, wobei dieses das Verschlusselement bildende Geflecht in einem in Gebrauchsstellung die Öffnung im Herzen durchsetzenden Mittelbereich radial eingeschnürt oder zusammengedrückt ist, so dass beidseits dieses eingeschnürten oder zusammengedrückten Bereichs geflochtene Verschlussscheiben oder -bereiche vorgesehen sind, die die Ränder der ungewollten Öffnung im Herzen in Gebrauchsstellung beidseits übergreifen.

Ein derartiges aus Nitinoldrähten geflochtenes Verschlusselement ist aus WO 99/12478 A1 bekannt. Ein ursprünglich etwa zylindrisches Geflecht ist zentral in radialer Richtung gestaucht und die beidseitigen Enden des Geflechts sind jeweils durch eine die einzelnen Drähte mit kleinerem Durchmesser erfassenden Hülsen oder Stutzen zusammengefasst und in der entsprechenden Form festgelegt, so dass beispielsweise bei einem zwischen den beiden Herzvorhöfen angeordnetes Verschlusselement auch im linken Vorhof, wo arterielles Blut einen relativ hohen Druck erzeugt, eine solche Hülse vorsteht und zu Störungen führen kann, wie sie in DE 10 338 702 B3, insbesondere in Absatz [0007], beschrieben sind. Aus dieser Druckschrift sind auch Ausführungsbeispiele bekannt, bei denen die Hülsen oder Stutzen in das Innere des Geflechts eingestülpt sind. Dadurch stehen sie zwar nicht mehr nach außen vor, jedoch hat das gesamte Verschlusselement auch durch diese in seinem Inneren nahe den Stirnseiten angeordneten Haltehülsen nur eine verhältnismäßig geringe Stabilität insbesondere in axialer Richtung.

Aus DE 10 338 702 B3 ist ein Verschlusselement der eingangs definierten Art bekannt, bei welchem nur eine derartige Hülse oder Fassung für die Enden der Drähte des Geflechts vorhanden ist und eine zweite derartige Hülse vermieden wird, damit dieses Verschlusselement so eingesetzt werden kann, dass im linken Vorhof des Herzens keine derartige Hülse vorhanden ist. Allerdings ist dabei die im linken Vorhof anzubringende bzw. in Gebrauchsstellung angeordnete geflochtene Verschlussscheibe nur einlagig, das heißt das etwas gewölbte oder scheibenförmig angeordnete Geflecht reicht von der inneren Zusammenschnürung bzw. dem Bereich kleineren Querschnitts nach außen und endet dort. Dies macht besondere Vorkehrungen erforderlich, damit diese nur einlagige Verschlussscheibe nicht durch den im linken Vorhof herrschenden höheren Blutdruck eventuell sogar durch die ungewollte Öffnung hindurchgedrückt werden kann. Beispielsweise muss ein entsprechend enges und demgemäß aufwendigeres Geflecht vorgesehen werden und/oder diese einlagige Verschlussscheibe muss überdimensioniert und größer ausgeführt werden, als es durch die ungewollte Öffnung im Herzen eigentlich erforderlich ist, oder es muss eine sonstige Verstärkung an dieser Verschlussscheibe vorgesehen werden.

Aus WO 01/21246 A1 ist ein aus einem Geflecht gebildetes Verschlusselement bekannt, bei welchem eine Stirnseite eingestülpt und ein Geflechtteil mit seinem Ende durch die andere Stirnseite hindurchgeführt ist, wobei an den Enden gegebenenfalls Ringe angeordnet und das eine Geflechtteil in dem distalen Ende des anderen Geflechtteils gehalten sein kann. Auch dies ergibt in axialer Richtung keine hohe Stabilität, weil die gegenseitige Lage der sich durchdringenden Geflechtteile nicht eindeutig und nicht schon vor der Benutzung fixiert ist.

Es besteht deshalb die Aufgabe, ein Verschlusselement der eingangs definierten Art zu schaffen, bei welchem der Vorteil erhalten bleibt, dass zumindest in dem linken Vorhof eines Herzens keine Hülse oder Fassung für die Fäden oder Drähte des Geflechts vorhanden ist, trotzdem aber die im linken Vorhof anzuordnende Verschlussscheibe eine ausreichende Steifigkeit oder Stabilität hat, um dem dort herrschenden höheren Blutdruck standhalten zu können, ohne beispielsweise überdimensioniert werden zu müssen. Außerdem soll das Verschlusselement eine gute Stabilisierung in axialer Richtung haben.

Zur Lösung dieser scheinbar widersprüchlichen Aufgabe ist das eingangs definierte Verschlusselement dadurch gekennzeichnet, dass das in Ausgangsstellung vor dem Einschnüren des Mittelbereichs etwa röhrenförmige Geflecht über seine gesamte axiale Erstreckung doppelwandig ausgeführt oder ausgebildet ist, also einen ersten Geflecht-Abschnitt mit geringerem Querschnitt und einen diesen außenseitig umschließenden zweiten Geflecht-Abschnitt mit größerem Querschnitt aufweist, wobei die beiden Geflecht-Abschnitte über die gesamte axiale Erstreckung des Verschlusselements verlaufen und an einer gemeinsamen Stirnseite ineinander übergehen oder sich die sie bildenden Fäden oder Drähte fortsetzen oder verbunden sind, so dass durch das Einschnüren oder Zusammendrücken eine vorsprungfreie, doppelwandige Verschlussscheibe gebildet ist, deren außenliegende Wand die gemeinsame Stirnseite umfasst bzw. von dieser gemeinsamen Stirnseite gebildet wird, dass der etwa röhrenförmige erste Geflecht-Abschnitt kleineren und insbesondere kreisförmigen Querschnitts an seinem freien Ende - in der Regel an dem Ende, wo das Flechten beginnt - eine Hülse oder eine Fassung aufweist und die Fäden oder Drähte des Geflechts durch diese Hülse oder Fassung festgelegt sind und dass der entgegengesetzt zu diesem ersten Geflecht-Abschnitt außenseitig verlaufende zweite Geflecht-Abschnitt größeren und insbesondere kreisförmigen Querschnitts mit seinen Fäden oder Drähten stirnseitig ebenfalls zu dieser Fassung oder Hülse zum Festlegen dieser Fäden oder Drähte in etwa radialer Richtung zusammengeführt ist.

Es wird also ein Geflecht verwendet, dessen Verlauf von einem inneren Abschnitt kleineren Durchmessers nach außen über eine stirnseitige Richtungsänderung fortgesetzt und in entgegengesetzter Richtung weitergeführt ist, so dass ein einziges, aber doppelwandiges Geflecht mit zwei konzentrischen Geflecht-Abschnitten das gesamte Verschlusselement weitgehend bildet und demgemäß an einer seiner Stirnseiten keine zusammenfassende Hülse für die Fäden oder Drähte erforderlich macht. Ferner ergibt sich dadurch eine Verdoppelung der Lagen des Geflechts im Bereich seiner radialen Vergrößerung von dem ersten inneren Geflechts-Abschnitt zu dem äußeren Geflechts-Abschnitt hin und von dort wieder zu der Einschnürung, so dass die dadurch gebildete Verschlussscheibe zumindest doppellagig ist.

Dadurch, dass das Geflecht zunächst in der einen axialen Richtung und dann an einer Stirnseite unter Vergrößerung des Querschnitts dieses Geflechts nach außen wieder zurückgeführt wird, entsteht zumindest in diesem stirnseitigen Bereich eine doppelwandige und doppelhülsenartige Struktur mit einer zunächst zentralen Öffnung durch den ersten inneren Geflecht-Abschnitt hindurch. Diese Öffnung kann durch die gemeinsame Hülse oder Fassung an dem Anfang dieses ersten Geflecht-Abschnitts in noch zu erläuternder Weise verschlossen werden, wobei diese Hülse beispielsweise etwa in der Art erzeugt oder angebracht werden kann, wie es aus DE 10 338 702 B3 bekannt ist.

Da durch einen sich in entgegengesetzten Richtungen fortsetzenden Flechtvorgang die dabei an der Flechtmaschine oder -vorrichtung befindliche Halterung der einzelnen Drähte immer wieder in ihre erste Ausgangslage oder sogar darüber hinaus gelangt, kann der gesamte Herstellungsvorgang solcher Verschlusselemente und dabei der Flechtvorgang erleichtert und rationalisiert werden und es ist sogar denkbar, nach Fertigstellung eines doppelwandigen Geflechts die an dessen Ende ankommenden Fäden oder Drähte zunächst ungeflochten in eine ursprüngliche Ausgangsstellung zurückzuführen und von dort aus wiederum das nächste doppelwandige Geflecht herzustellen, so dass eine Serienfertigung derartiger Verschlusselemente durch die erfindungsgemäße doppelwandige Anordnung von miteinander verbundenen Geflecht-Abschnitten ermöglicht wird, ohne dass die Flechtvorrichtung nach Fertigstellung eines jeden Verschlusselements neu eingerichtet werden muss, wie es bei der Lösung gemäß DE 10 338 702 B3 erforderlich ist. Somit hat das erfindungsgemäße Verschlusselement trotz des scheinbar größeren Aufwandes eines doppelwandigen Geflechts und trotz des für die Verschlussfunktion günstigen, scheinbar größeren Materialverbrauchs den zusätzlichen Vorteil, eine günstige und preiswerte Serienfertigung zu erlauben. Gleichzeitig wird die Sicherheit des Verschlusselements auch im linken Vorhof eines Herzens vergrößert.

Das Verschlusselement ist besonders vorteilhaft als ASD-Verschluss, kann aber auch als PDA- oder als VSD-Verschluss benutzt werden, wobei ebenfalls der Vorteil genutzt wird, dass an einer Stirnseite des Verschlusselements keine Haltehülse für die Fäden oder Drähte vorsteht und beide Verschlussscheiben doppellagig sein können und eine etwa übereinstimmende Festigkeit und große Stabilität auch in axialer Richtung haben.

Dabei kommt man mit nur einer Hülse oder Fassung für die Fäden oder Drähte des gesamten Geflechts aus, wobei dann allerdings der erste Geflecht-Abschnitt eine entsprechend größere, über die gesamte axiale Erstreckung des Verschlusselements reichende Länge schon in Ausgangslage vor dem Einschnüren und Stauchen dieses Verschlusselements hat und mitgestaucht wird.

Zweckmäßig ist es dabei, wenn zumindest der dem ersten inneren Geflecht-Abschnitt über die gemeinsame Stirnseite und eine Verschlussscheibe fortsetzende und außen insbesondere koaxial und konzentrisch und mit Abstand übergreifende zweite Geflecht-Abschnitt in einem mittleren axialen Bereich eingeschnürt und das gesamte Verschlusselement in axialer Richtung gestaucht ist. Dies ergibt beidseits der eingeschnürten Stelle die erwünschten Verschlussscheiben, wobei deren axiale Abmessung durch die Stauchung gering gehalten werden kann, gleichzeitig aber auch eine ausreichend große radiale Erstreckung zum Übergreifen der Ränder der Öffnung im Herzen erzielt wird.

Der Durchmesser des zweiten Geflecht-Abschnitts kann vor der Einschnürung etwa fünfmal bis fünfzehnmal, insbesondere etwa zehnmal, größer als der des ersten Geflecht-Abschnitts sein. Dadurch wird erreicht, dass vor allem die Verschlussscheiben überwiegend durch den größeren zweiten Geflecht-Abschnitt gebildet werden, die dann aber beide mindestens zweilagig sind.

Günstig ist es dabei, wenn in Gebrauchsstellung der innenliegende und der außenliegende Geflecht-Abschnitt beidseits der Einschnürung in axialer Richtung gestaucht und dadurch in radialer Richtung vergrößert sind und die so gebildeten beiden Verschlussscheiben zumindest in ihrem Mittelteil oder Zentrumsbereich in axialer Richtung gesehen insgesamt vierlagig und im radialen äußeren Bereich außerhalb des innenliegenden Geflecht-Abschnitts zweilagig sind. Wird ein Geflecht, bei welchem der erste und der zweite Geflecht-Abschnitt zunächst über die gesamte axiale Erstreckung reicht, gestaucht, werden beide Geflecht-Abschnitte gestaucht, wobei der innenliegende Geflecht-Abschnitt aufgrund seiner geringeren radialen Ausdehnung zu entsprechend kleineren scheibenförmigen Gebilden führt, während der zweite Geflecht-Abschnitt aufgrund seiner erheblich größeren radialen Ausdehnung in gestauchtem Zustand auch zu entsprechend erheblich größeren Verschlussscheiben führt, die die wesentlichen Teile des Verschlusselements bilden.

Die die Fäden oder Drähte zusammenfassende Hülse oder Fassung kann an ihrer axial nach außen weisenden Stirnseite oder Stirnfläche ein Innengewinde für einen Zuführkatheder oder für ein Manipulierwerkzeug haben. Dadurch kann die einzige Hülse in vorteilhafter Weise zum Zuführen oder Manipulieren der Vorrichtung nutzbar gemacht werden.

Vor allem bei Kombination einzelner oder mehrerer der vorbeschriebenen Merkmale und Maßnahmen ergibt sich ein Verschlusselement, bei welchem beidseits einer ungewollten Öffnung angeordnete Verschlussscheiben mindestens zweilagig sind, so dass man auch mit dünneren Fäden oder Drähten arbeiten kann, die demgemäß weniger steif sind und deshalb der Anatomie beispielsweise im Herzen entgegenkommen und auch bei der Herstellung sowie später beim Einbringen des Verschlusselements mit Hilfe eines Zuführkatheders die erforderlichen Verformungen vereinfachen, wobei nur eine einzige Hülse oder Fassung zum Festlegen der Fäden oder Drähte erforderlich ist.

Nachstehend ist ein Ausführungsbeispiel der Erfindung anhand der Zeichnung näher beschrieben. Es zeigt in zum Teil schematisierter Darstellung:
- Fig. 1: eine Ansicht eines Verschlusselements bestehend aus einem ersten innenliegenden Geflecht-Abschnitt klei- neren Durchmessers, der an einer Stirnseite über die ihn bildenden Drähte oder Fäden in einen außenliegen- den zweiten Geflecht-Abschnitt größeren Durchmessers übergeht, wobei beide Geflecht-Abschnitte koaxial und konzentrisch zueinander angeordnet und an der der ge- meinsamen Stirnseite entgegengesetzten Stirnseite eine gemeinsame Hülse oder Fassung für die sie bil- denden Drähte oder Fäden hat,
- Fig. 2: in schematisierter Andeutung die Einschnürung zu- nächst des zweiten außenliegenden Geflecht-Ab- schnitts,
- Fig. 3: eine Ansicht des Verschluss-Elements nach dem Ein- schnüren und Stauchen in axialer Richtung, wobei die Fäden und Geflecht-Teile in axialer Richtung noch über die gemeinsame Hülse oder Fassung überstehen, sowie
- Fig. 4: das fertiggestellte Verschlusselement mit einer ge- meinsamen an einer Stirnseite des Verschlusselements überstehenden Hülse zum Zusammenhalten der Fäden oder Drähte, während an der abgewandten Stirnseite keine derartige Hülse übersteht und beide Verschlussschei- ben dieses Verschlusselements auch in ihren radial außenliegenden Bereichen zumindest doppelwandig sind.

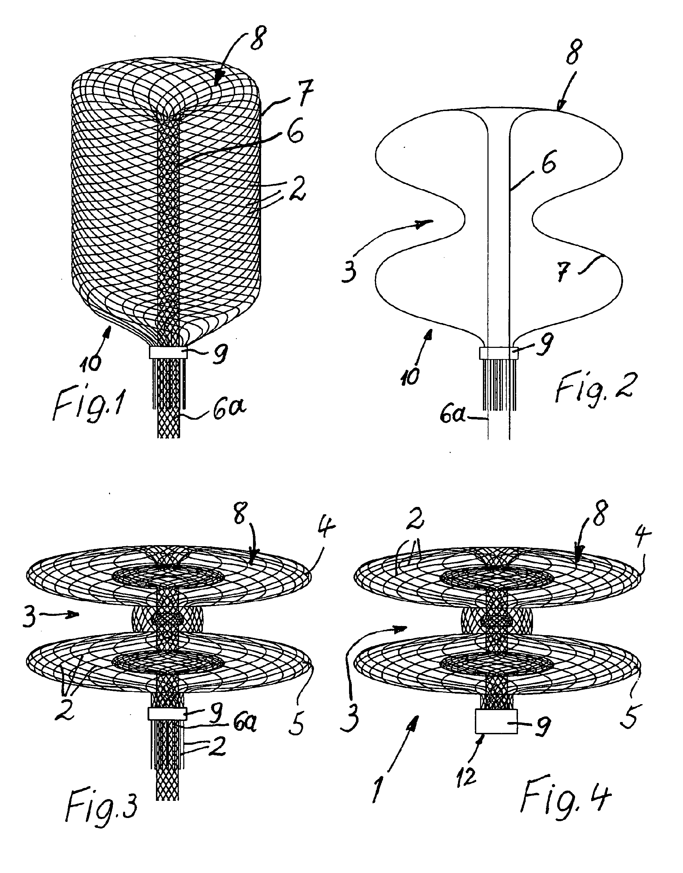

Ein im Ganzen mit 1 bezeichnetes, vor allem in Fig. 4 dargestelltes Verschlusselement ist zum Einsetzen in ungewollte Öffnungen im Herzen als ASD-Verschlusselement oder Occlusionsinstrument gedacht, könnte aber auch als PDA- oder VSD-Verschluss benutzt werden.

Dieses Verschlusselement 1 ist aus Memory-Materialfäden 2, beispielsweise aus Nitinolfäden oder aus mit Kunststoff ummantelten Drähten oder Fäden oder auch aus Memory-Kunststofffäden geflochten, was besonders gut in Fig. 1, aber auch in Fig. 3 und 4 erkennbar ist.

Dabei ist dieses das Verschlusselement 1 bildende, besonders gut in Fig. 1 erkennbare Geflecht in einem in Gebrauchsstellung die Öffnung im Herzen durchsetzenden Mittelbereich 3 gemäß den Fig. 2 bis 4 radial eingeschnürt oder zusammengedrückt, so dass beidseits dieses eingeschnürten oder zusammengedrückten Mittelbereichs 3 geflochtene Verschlussscheiben 4 und 5 oder -bereiche gebildet sind, die die Ränder der ungewollten Öffnung im Herzen in Gebrauchsstellung beidseits übergreifen, wie es beispielsweise aus Fig. 16 von US-A-5 725 552 und ferner aus DE 10 338 702 B3 bekannt ist.
Dabei ist gemäß Fig. 1 vorgesehen, dass das in Ausgangsstellung vor dem Einschnüren des Mittelbereichs 3 etwa röhrenförmige Geflecht über die gesamte axiale Erstreckung doppelwandig ausgebildet ist, also einen ersten Geflecht-Abschnitt 6 mit geringerem Querschnitt und einen diesen außenseitig umschließenden oder übergreifenden zweiten Geflecht-Abschnitt 7 mit größerem Querschnitt aufweist.

Dabei erkennt man vor allem in Fig. 1, aber auch in den weiteren Figuren deutlich, dass die beiden Geflecht-Abschnitte 6 und 7 an einer gemeinsamen Stirnseite 8 ineinander übergehen oder sich fortsetzen oder verbunden sind, das heißt die Fäden oder Drähte 2 gehen einstückig von dem ersten Geflecht-Abschnitt 6 in den zweiten Geflecht-Abschnitt 7 größeren Durchmessers über, wobei dieser radiale Übergang die Stirnseite 8 des gesamten Geflechts bzw. des Verschlusselements 1 bildet.

Dadurch ergibt sich, dass durch das Einschnüren oder Zusammendrücken des Mittelbereichs 3 eine vorsprungfreie, wenigstens doppelwandige Verschlussscheibe 4 gebildet wird, deren außenliegende Wand die gemeinsame Stirnseite 8 umfasst oder dieser Stirnseite 8 entspricht, wie es vor allem in den Fig. 2 bis 4 angedeutet ist.

Gleichzeitig erkennt man in den Figuren, dass an dieser Verschlussscheibe 4 kein axial nach außen ragender oder vorstehender Vorsprung mit einer Fassung oder Hülse für die Fäden oder Drähte 2 vorhanden ist.

Gemäß Fig. 3 ist zumindest der den ersten inneren Geflecht-Abschnitt 6 über die gemeinsame Stirnseite 8 und eine Verschlussscheibe 4 fortsetzende und außen koaxial und mit Abstand übergreifende zweite Geflecht-Abschnitt 7 in dem axialen Mittelbereich 3 eingeschnürt und gemäß den Fig. 3 und 4 das gesamte Verschlusselement 1 in axialer Richtung gestaucht, so dass die ursprünglich gemäß Fig. 1 vorgesehene Abmessung der axialen Erstreckung bei dem fertigen Verschlusselement 1 erheblich geringer ist, während gleichzeitig seine radiale Abmessung oder Ausdehnung gegenüber dem Ausgangs-Rohling vergrößert ist. Dabei haben die beiden Verschlussscheiben 4 und 5 etwa übereinstimmende Abmessungen.

Dabei erkennt man, dass der Durchmesser des zweiten Geflecht-Abschnitts 7 vor der Einschnürung etwa fünfmal bis fünfzehnmal, im Ausführungsbeispiel etwa zehnmal so groß wie der des ersten Geflecht-Abschnitts 6 ist, das heißt vor allem der zweite Geflecht-Abschnitt 7 bildet in Gebrauchsstellung die Verschlussscheiben 4 und 5 aus, wobei aber der erste Geflecht-Abschnitt 6 die wichtige Aufgabe hat, die Fäden oder Drähte 2 des Verschlusselements 1 zusammenzuhalten und zu erfassen, ohne dass an der Stirnseite 8 und damit der Verschlussscheibe 4 in axialer Richtung ein Vorsprung mit einer Haltehülse erforderlich ist.

Erreicht wird dies dadurch, dass der etwa röhrenförmige erste Geflecht-Abschnitt 6 kleineren und insbesondere kreisförmigen Querschnitts an seinem freien Ende 6a, von wo der Flechtvorgang zur Bildung des Geflechts ausgeht, eine Hülse 9 oder Fassung, im Folgenden auch nur "Hülse 9" genannt, aufweist und die Fäden oder Drähte 2 des Geflechts durch diese Hülse 9 festgelegt sind, wobei die Querschnitts- oder Durchmesserabmessung dieser Hülse 9 die des Geflecht-Abschnitts 6 nicht oder nur wenig übertrifft und diese Hülse 9 auch als vollständig ausgefüllter Stutzen analog der Ausführung gemäß DE 10 338 702 B3 ausgeführt sein kann.

Der entgegengesetzt zu diesem ersten Geflecht-Abschnitt 6 außenseitig verlaufende zweite Geflecht-Abschnitt 7 größeren und insbesondere kreisförmigen Querschnitts ist mit seinen Fäden oder Drähten 2 stirnseitig, also an der der Stirnseite 8 entgegengesetzten Stirnseite 10, ebenfalls zu einer Fassung oder Hülse zum Festlegen dieser Fäden oder Drähte 2 in etwa radialer Richtung zusammengeführt, so dass alle Fäden oder Drähte 2 des Geflechts so fixiert sind, dass sich das Geflecht nicht auflösen kann, wobei die Hülse mit den Fäden oder Drähten einen ausgefüllten Stutzen bilden könnte.

Nach Fig. 1 ist dabei vorgesehen, dass die Hülse 9 für die Fäden oder Drähte 2 des ersten Geflecht-Abschnitts 6 auch die Fäden oder Drähte 2 des zweiten Geflecht-Abschnitts 7 enthält, zusammenhält oder aufweist und dass beide Geflecht-Abschnitte 6 und 7 über die gesamte axiale Erstreckung des Verschlusselements 1 verlaufen. Man könnte auch sagen, dass die Hülsen zum Zusammenfassen der Drähte 2 des ersten Geflecht-Abschnitts 6 und zum Zusammenfassen der Drähte 2 des zweiten Geflecht-Abschnitts 7 einstückig zu einer einzigen Hülse 9 verbunden sind.

Durch diese Anordnung ergibt sich ein Verschlusselement 1 gemäß den Fig. 3 und 4, bei welchen in Gebrauchsstellung der innenliegende und der außenliegende Geflecht-Abschnitt 6 und 7 beidseits der Einschnürung im Mittelbereich 3 in axialer Richtung gestaucht und dadurch in radialer Richtung vergrößert sind und die so gebildeten beiden Verschlussscheiben 4 und 5 in Ihrem Zentrumsbereich in axialer Richtung gesehen insgesamt vierlagig und im radial äußeren Bereich außerhalb des innenliegenden Geflecht-Abschnitts 6 zweilagig sind. Durch die Stauchung gemäß Fig. 3 und 4 wird nämlich auch der innenliegende Geflecht-Abschnitt 6 entsprechend gestaucht, was allerdings an der Einschnürung im Mittelbereich 3 weitgehend verhindert ist, so dass die axiale Verkürzung sowohl des innenliegenden ersten Geflecht-Abschnitts 6 als auch die des außenliegenden zweiten Geflecht-Abschnitts 7 an diesen Geflecht-Abschnitten 6 und 7 zu entsprechenden radialen Aufweitungen und damit zur Ausbildung der erwähnten Verschlussscheiben 4 und 5 führt.

In nicht näher dargestellter Weise kann die gemeinsame Hülse 9 an ihrer axial nach außen zu der Stirnseite 10 hinweisenden Stirnseite oder Stirnfläche 12 ein Innengewinde für einen Zuführkatheder bzw. für ein zum Verschieben und Positionieren des Verschlusselements 1 dienendes Manipulierwerkzeug haben.

Das Verschlusselement 1 für ungewollte Öffnungen im Herzen, welches vor allem als ASD-Verschlusselement oder Occlusionsinstrument dienen kann, ist aus Memory-Material-Fäden 2, beispielsweise aus Kunststofffäden, Nitinoldrähten oder aus mit Kunststoff ummantelten Drähten oder Fäden aus Memory-Material geflochten. Dieses das Verschlusselement 1 bildende Geflecht ist in einem in Gebrauchsstellung die Öffnung im Herzen durchsetzenden Mittelbereich 3 radial eingeschnürt oder zusammengedrückt oder geringer bemessen, so dass beidseits dieses eingeschnürten, zusammengedrückten oder geringer bemessenen Mittelbereichs 3 geflochtene Verschlussscheiben 4 und 5 oder Verschlussbereiche vorgesehen sind, die die Ränder einer ungewollten Öffnung im Herzen in Gebrauchsstellung beidseits übergreifen. Dabei ist in Ausgangsstellung vor dem Einschnüren des Mittelbereichs 3 ein röhrenförmiges Geflecht über mindestens einen Teil seiner axialen Erstreckung doppelwandig ausgebildet, das heißt es ist ein erster Geflecht-Abschnitt 6 mit geringerem Querschnitt und ein diesen außenseitig mit Abstand umschließender zweiter Geflecht-Abschnitt 7 mit größerem Querschnitt vorgesehen, wobei die Fäden oder Drähte 2 der beiden Geflecht-Abschnitte 6 und 7 an einer gemeinsamen Stirnseite 8 einander fortsetzen oder einstückig verbunden sind, so dass das durch das Einschnüren oder Zusammendrücken wenigstens eine vorsprungfreie, doppelwandige Verschlussscheibe 4 gebildet ist, deren außenliegende Wand durch die gemeinsame Stirnseite 8 gebildet ist oder diese umfasst.

## Patentansprüche

1. Verschlusselement (1) für ungewollte Öffnungen im Herzen, insbesondere Verschlusselement für Vorhofseptum-Defekt (ASD) oder Occlusionsinstrument, welches aus Memory-Material-Fäden (2), insbesondere aus Memory-Kunststofffäden, aus Nitinoldrähten oder aus mit Kunststoff ummantelten Drähten oder Fäden aus Memory-Material geflochten ist, wobei dieses das Verschlusselement (1) bildende Geflecht in einem in Gebrauchsstellung die Öffnung im Herzen durchsetzenden Mittelbereich (3) radial eingeschnürt oder zusammengedrückt ist, so dass beidseits dieses eingeschnürten oder zusammengedrückten Mittelbereichs (3) geflochtene Verschlussscheiben (4,5)oder -bereiche vorgesehen sind, die die Ränder der ungewollten Öffnung im Herzen in Gebrauchsstellung beidseits übergreifen, **dadurch gekennzeichnet, dass** das in Ausgangsstellung vor dem Einschnüren des Mittelbereichs (3)etwa röhrenförmige Geflecht über seine gesamte axiale Erstreckung doppelwandig ausgebildet ist, also einen ersten Geflecht-Abschnitt (6) mit geringerem Querschnitt und einen diesen außenseitig umschließenden zweiten Geflecht-Abschnitt (7) mit größerem Querschnitt aufweist, wobei die beiden Geflecht-Abschnitte (6, 7) über die gesamte axiale Erstreckung des Verschlusselements (1) verlaufen und an einer gemeinsamen Stirnseite (8) ineinander übergehen oder sich fortsetzen oder verbunden sind, so dass durch das Einschnüren oder Zusammendrücken eine vorsprungfreie, doppelwandige Verschlussscheibe (4) gebildet ist, deren außenliegende Wand die gemeinsame Stirnseite (8) umfasst, dass der etwa röhrenförmige erste Geflecht-Abschnitt (6) kleineren Querschnitts an seinem freien Ende (6a) eine Hülse (9) oder eine Fassung aufweist und die Fäden oder Drähte (2)des Geflechts durch diese Hülse (9) oder Fassung festgelegt sind und dass der entgegengesetzt zu diesem ersten Geflecht-Abschnitt (6) außenseitig verlaufende zweite Geflecht-Abschnitt (7) größeren Querschnitts mit seinen Fäden oder Drähten (2) stirnseitig ebenfalls zu dieser Fassung oder Hülse (9) zum Festlegung dieser Fäden oder Drähte (2) in etwa radialer Richtung zusammengeführt ist.

2. Verschlusselement nach Anspruch 1, **dadurch gekennzeichnet, dass** zumindest der den ersten inneren Geflecht-Abschnitt (6)über die gemeinsame Stirnseite (8) und eine Verschlussscheibe (4) fortsetzende und außen insbesondere koaxial und mit Abstand übergreifende zweite Geflecht-Abschnitt (7) in dem axialen Mittelbereich (3) eingeschnürt und das gesamte Verschlusselement (1) in axialer Richtung gestaucht ist.

3. Verschlusselement nach Anspruch 1 oder 2, **dadurch gekennzeichnet, dass** der Durchmesser des zweiten Geflecht-Abschnitts (7) vor der Einschnürung etwa fünfmal bis fünfzehnmal, insbesondere etwa zehnmal, größer als der des ersten Geflecht-Abschnitts (6) ist.

4. Verschlusselement nach einem der Ansprüche 1 bis 3, **dadurch gekennzeichnet, dass** der erste Geflecht-Abschnitt (6) und der zweite Geflecht-Abschnitt (7) einen kreisförmigen Querschnitt haben.

5. Verschlusselement nach einem der Ansprüche 1 bis 4, **dadurch gekennzeichnet, dass** in Gebrauchsstellung der innenliegende und der außenliegende Geflecht-Abschnitt (6, 7) beidseits der Einschnürung (3) in axialer Richtung gestaucht und **dadurch** in radialer Richtung vergrößert sind und die so gebildeten beiden Verschlussscheiben (4, 5) zumindest in ihrem Mittelteil oder Zentrumsbereich in axialer Richtung gesehen insgesamt vierlagig und im radialen äußeren Bereich außerhalb des innenliegenden Geflecht-Abschnitts (6) zweilagig sind.

6. Verschlusselement nach einem der Ansprüche 1 bis 5, **dadurch gekennzeichnet, dass** die die Fäden oder Drähte (2) zusammenfassende Hülse (9) oder Fassung an ihrer axial nach außen weisenden Stirnseite oder Stirnfläche (12) ein Innengewinde oder eine Angriffsstelle für einen Zuführkatheter oder ein Manipulierwerkzeug hat.

## Claims

1. Closure element (1) for undesirable openings in the heart, particularly a closure element for Atrial Septal Defect (ASD) or occlusion instrument which is woven from memory material filaments (2), particularly from plastic memory filaments, from nitinol wires or from plastic-coated wires or filaments of memory material, this meshwork that forms the closure element (1) being radially constricted or compressed in a central region (3) that passes through the opening in the heart, so that on both sides of this constricted or compressed central region (3) woven closure discs (4, 5) or closure regions are provided which overlap the edges of the undesirable opening in the heart on both sides in the position of use, **characterised in that** the meshwork which is substantially tubular in the original position before the central region (3) is constricted is of double-walled construction over its entire axial extent, i.e, it has a first meshwork portion (6) of smaller cross-section and a second meshwork portion (7) of larger cross-section that externally surrounds the latter, the two meshwork portions (6, 7) extending over the entire axial extent of the closure element (1) and merging with one another or continuing or being connected at a common end face (8), so that the constriction or compression produces a double-walled closure disc (4) free from projections, the outer wall of which surrounds the common end face (8), **in that** the substantially tubular first meshwork portion (6) of smaller cross-section comprises at its free end (6a) a sleeve (9) or a socket, and the filaments or wires (2) of the meshwork are secured by this sleeve (9) or socket, and **in that** the second meshwork portion (7) of larger cross-section extending on the outside opposite this first meshwork portion (6) also converges in a substantially radial direction with its filaments or wires (2) on the end face towards this socket or sleeve (9) for securing these filaments or wires (2).

2. Closure element according to claim 1, **characterised in that** at least the second meshwork portion (7), which continues the first inner meshwork portion (6) over the common end face (8) and a closure disc (4) and overlaps them externally, in particular coaxially and at a spacing, is constricted in the axial central region (3) and the entire closure element (1) is compressed in the axial direction.

3. Closure element according to claim 1 or 2, **characterised in that** the diameter of the second meshwork portion (7) before the constriction is about five to fifteen times, more particularly about ten times, greater than that of the first meshwork portion (6).

4. Closure element according to one of claims 1 to 3, **characterised in that** the first meshwork portion (6) and the second meshwork portion (7) have a circular cross-section.

5. Closure element according to one of claims 1 to 4, **characterised in that** in the position of use the inner and outer meshwork portions (6, 7) are compressed on both sides of the constriction (3) in the axial direction and thereby enlarged in the radial direction and the two closure discs (4, 5) thus formed are made up of a total of four layers in the axial direction, at least in their central part or in the region of the centre, and of two layers in the radial outer region outside the inner meshwork portion (6).

6. Closure element according to one of claims 1 to 5, **characterised in that** the sleeve (9) or socket that holds the filaments or wires (2) together has, on its axially outward facing end or end face (12), an internal thread or a point of engagement for a supply catheter or a manipulating tool.

## Revendications

1. Elément (1) d'obturation d'orifices indésirables présents dans le coeur, notamment élément d'obturation assigné à une défaillance du septum auriculaire (ASD) ou instrument d'occlusion tressé à partir de fils (2) en un matériau à mémoire de forme, en particulier des fils en matière plastique à mémoire de forme, des fils métalliques en Nitinol, voire des fils métalliques ou des fils en un matériau à mémoire de forme, enrobés de matière plastique, sachant que cette structure tressée, constituant l'élément d'obturation (1), est resserrée ou comprimée radialement dans une région centrale (3) qui, en position d'utilisation, traverse l'orifice présent dans le coeur de façon telle qu'il soit prévu, de part et d'autre de cette région centrale (3) resserrée ou comprimée, des disques obturateurs (4, 5) ou des régions obturatrices tressé(e)s venant coiffer de part et d'autre, en position d'utilisation, les bords de l'orifice indésirable présent dans le coeur, **caractérisé par le fait que** ladite structure tressée, sensiblement tubulaire en position initiale préalablement au resserrement de ladite région centrale (3), est de réalisation à double paroi sur toute son étendue axiale, c'est-à-dire qu'elle comprend une première zone tressée (6) de section transversale moindre et une seconde zone tressée (7) de plus grande section transversale, entourant extérieurement la zone précitée, sachant que les deux zones tressées (6, 7) couvrent toute l'étendue axiale dudit élément d'obturation (1) et fusionnent l'une dans l'autre ou se prolongent, voire sont solidarisées au niveau d'une face extrême commune (8), si bien que le resserrement ou la contraction donne naissance à un disque obturateur (4) à paroi double, exempt d'excroissances, dont la paroi extérieure englobe ladite face extrême commune (8) ; **par le fait que** ladite première zone tressée (6) sensiblement tubulaire, de section transversale plus petite, présente une douille (9) ou une monture à son extrémité libre (6a), les fils ou fils métalliques (2) de ladite structure tressée étant verrouillés à demeure par cette douille (9) ou cette monture ; et **par le fait que** ladite seconde zone tressée (7) de section transversale plus grande, s'étendant extérieurement en sens inverse de cette première zone tressée (6), est pareillement regroupée vers cette monture ou cette douille (9) à sa face extrême, par ses fils ou fils métalliques (2), en vue du verrouillage à demeure de ces fils ou fils métalliques (2) dans une direction sensiblement radiale.

2. Elément d'obturation selon la revendication 1, **caractérisé par le fait qu'**au moins la seconde zone tressée (7) prolongeant la première zone tressée intérieure (6) par la face extrême commune (8) et par un disque obturateur (4), et coiffant extérieurement ladite zone, en particulier coaxialement et à distance, est resserrée dans la région centrale axiale (3), ledit élément d'obturation (1) étant intégralement refoulé dans le sens axial.

3. Elément d'obturation selon la revendication 1 ou 2, **caractérisé par le fait que**, préalablement au resserrement, le diamètre de la seconde zone tressée (7) est d'environ cinq fois à quinze fois, notamment environ dix fois supérieur à celui de la première zone tressée (6).

4. Elément d'obturation selon l'une des revendications 1 à 3, **caractérisé par le fait que** la première zone tressée (6) et la seconde zone tressée (7) offrent une section transversale circulaire.

5. Elément d'obturation selon l'une des revendications 1 à 4, **caractérisé par le fait que**, dans la position d'utilisation, les zones tressées (6, 7) intérieure et extérieure sont refoulées dans le sens axial, de part et d'autre du resserrement (3), en étant par conséquent agrandies dans le sens radial ; et, considérés dans le sens axial, les deux disques obturateurs (4, 5) ainsi formés comptent globalement quatre couches, au moins dans leur partie médiane ou leur région centrale, et deux couches dans la région radiale extérieure, en dehors de la zone tressée intérieure (6).

6. Elément d'obturation selon l'une des revendications 1 à 5, **caractérisé par le fait que** la douille (9) ou la monture regroupant les fils ou fils métalliques (2) comporte, au niveau de sa face extrême ou surface frontale (12) pointant vers l'extérieur dans le sens axial, un filetage intérieur ou une zone offrant prise à un cathéter d'amenée ou à un instrument de manipulation.
